# EUROPEAN PATENT APPLICATION

(11) **EP 3 725 882 A1**
(43) Date of publication of application: **21.10.2020**
(21) Application number: 18888764.0
(22) Date of filing: 14.12.2018
(51) Int. Cl.: C12N 15/113, C12Q 1/6809

(54) **METHOD FOR AIDING DETECTION OF ALZHEIMER'S DISEASE**

(30) Priority: 14.12.2017 JP 2017239961
(71) Applicant: Hiroshima University, Higashihiroshima-shi Hiroshima 739-8511 (JP)
(72) Inventor: TAHARA, Hidetoshi, Hiroshima-shi, Hiroshima 734-8553 (JP)
(74) Representative: Mewburn Ellis LLP
(86) International application number: PCT/JP2018/046198
(87) International publication number: WO 2019/117306

(57) **Abstract**

Disclosed is a method of assisting the detection of Alzheimer's disease, assisting in highly accurate detection of Alzheimer's disease. In the method of assisting the detection of Alzheimer's disease, the abundance of at least one of miRNAs or the like contained in a test sample isolated from a living body, whose nucleotide sequence is represented by any one of SEQ ID NOs: 1 to 85, is used as an index. A higher abundance of at least one of the miRNAs or the like whose nucleotide sequence is represented by any one of SEQ ID NOs: 1 to 22 and 66 to 71 than that of healthy subjects or a lower abundance of at least one of the miRNAs or the like whose nucleotide sequence is represented by any one of SEQ ID NOs: 23 to 65 and 72 to 85 than that of healthy subjects indicates a higher likelihood of having Alzheimer's disease.

## Description

### TECHNICAL FIELD

The present invention relates to a method of assisting the detection of Alzheimer's disease.

### BACKGROUND ART

Alzheimer's disease is the most common type of dementia, which accounts for more than half of all dementia cases, and the number of patients with this disease is expected to increase more in future aging societies. In current medicine, drugs and treatments to slow down the progression of Alzheimer's disease, a progressive disease, are available, though it is impossible to fully cure or prevent the progression of the disease. Thus, it is desirable to detect Alzheimer's disease as early as possible. Although Alzheimer's disease is currently diagnosed by means of medical interview, brain MRI scanning, and the like, it is not an easy task to detect early Alzheimer's disease.

Methods in which the abundance of microRNA (hereinafter referred to as "miRNA") in blood is used as an index to detect Alzheimer's disease at an early stage have been proposed (Patent Documents 1 to 4, Non-Patent Documents 1 to 3).

### PRIOR ART DOCUMENTS

### Patent Documents

Patent Document 1: JP 2014-132863 A
Patent Document 2: JP 2014-520529 T
Patent Document 3: EP 2733219 A1
Patent Document 4: WO 2016/148073

### Non-Patent Documents

Non-Patent Document 1: Pavan Kumar et al., PLOS ONE, July 2013, Volume 8, Issue 7, e69807, pp.1-10.
Non-Patent Document 2: Petra Leindinger et al., Genome Biology 2013, 14: R78.
Non-Patent Document 3: Wang-Xia Wang et al., The Journal of Neuroscience, January 30, 2008, 28(5): 1213-1223.

### SUMMARY OF THE INVENTION

### PROBLEM TO BE SOLVED BY THE INVENTION

As described above, various miRNAs have been proposed as indexes for the detection of Alzheimer's disease and, needless to say, it is advantageous if Alzheimer's disease can be detected with higher accuracy.

Thus, an object of the present invention is to provide a method of assisting the detection of Alzheimer's disease which assists in highly accurate detection of Alzheimer's disease.

### MEANS FOR SOLVING THE PROBLEM

As a result of intensive study, the inventors newly found miRNAs, isoform miRNAs (isomiRs), non-coding RNAs (ncRNAs), transfer RNA fragments (tRFs), LincRNAs, and MiscRNAs which increase or decrease in abundance in Alzheimer's disease, and discovered that use of these as indexes enables highly accurate detection of Alzheimer's disease, to thereby complete the present invention.

That is, the present invention provides the following:
(1) A method of assisting the detection of Alzheimer's disease, using as an index the abundance of at least one of miRNAs, isomiRs, precursor miRNAs, ncRNAs, transfer RNA fragments, LincRNAs, or MiscRNAs contained in a test sample isolated from a living body, whose nucleotide sequence is represented by any one of SEQ ID NOs: 1, 3, 12, 2, 4 to 11, and 13 to 85, wherein a higher abundance of at least one of the miRNAs, isomiRs, precursor miRNAs, ncRNAs, transfer RNA fragments, LincRNAs, or MiscRNAs whose nucleotide sequence is represented by any one of SEQ ID NOs: 1 to 22 and 66 to 71 than that of healthy subjects or a lower abundance of at least one of the miRNAs, isomiRs, precursor miRNAs, ncRNAs, transfer RNA fragments, LincRNAs, or MiscRNAs whose nucleotide sequence is represented by any one of SEQ ID NOs: 23 to 65 and 72 to 85 than that of healthy subjects indicates a higher likelihood of having Alzheimer's disease.
(2) The method according to (1), wherein the abundance of at least one of miRNAs, isomiRs, precursor miRNAs, ncRNAs, transfer RNA fragments, LincRNAs, or MiscRNAs whose nucleotide sequence is represented by any one of SEQ ID NOs: 1, 3, 12, 2, 4 to 11, and 13 to 65 is used as an index.
(3) The method according to (1), wherein the abundance of at least one of miRNAs, isomiRs, precursor miRNAs, transfer RNA fragments, LincRNAs, or MiscRNAs whose nucleotide sequence is represented by any one of SEQ ID NOs: 1, 3, 12, 2, 4 to 11, 46 to 65, 84, and 85 is used as an index.
(4) The method according to (3), wherein the abundance of at least one of miRNAs, isomiRs, precursor miRNAs, transfer RNA fragments, or MiscRNAs whose nucleotide sequence is represented by any one of SEQ ID NOs: 1, 3, 12, 2, 4 to 11, and 46 to 65 is used as an index.
(5) The method according to (1), wherein the abundance of at least one of miRNAs, isomiRs, or precursor miRNAs whose nucleotide sequence is represented by any one of SEQ ID NOs: 1, 3, 12, and 11 is used as an index.

### EFFECT OF THE INVENTION

By the method of the present invention, Alzheimer's disease can be highly accurately and yet conveniently detected. Thus, the method of the present invention will greatly contribute to the detection of Alzheimer's disease.

### DETAILED DESCRIPTION OF THE INVENTION

As described above, the abundance of a particular molecule selected from miRNAs, isomiRs, precursor miRNAs, ncRNAs, transfer RNA fragments, LincRNAs, or MiscRNAs (hereinafter sometimes referred to as "miRNAs or the like" for convenience) contained in a test sample isolated from a living body is used as an index in the method of the present invention. These miRNAs or the like (for example, "a miRNA or the like whose nucleotide sequence is represented by SEQ ID NO: 1" is hereinafter sometimes referred to simply as "a miRNA or the like represented by SEQ ID NO: 1" or "one represented by SEQ ID NO: 1" for convenience) themselves are known, and the nucleotide sequences thereof are as shown in Sequence Listing. The list of miRNAs or the like used in the method of the present invention is presented in Table 1.

**Table 1-1**

| SEQ ID NO: | Class | Archetype | Type | Length (nucleotides) | Sequence |
|---|---|---|---|---|---|
| 1 | miRNA | mir-340 | Mature 3' | 22 | uccgucucaguuacuuuauagc |
| 2 | miRNA | mir-122 | Mature 5' | 22 | uggagugugacaaugguguuug |
| 3 | isomiR | mir-181b-1//mir-181b-2 | Mature 5' sub | 22 | acauucauugcugucggugggu |
| 4 | isomiR | mir-451a | Mature 5' super | 24 | aaaccguuaccauuacugaguuua |
| 5 | isomiR | mir-20a | Mature 5' sub | 22 | uaaagugcuuauagugcaggua |
| 6 | miRNA | mir-20a | Mature 5' | 23 | uaaagugcuuauagugcagguag |
| 7 | isomiR | mir-20a | Mature 5' sub | 21 | uaaagugcuuauagugcaggu |
| 8 | isomiR | mir-451 a | Mature 5' super | 23 | aaaccguuaccauuacugaguuu |
| 9 | MiscRNA | ENST00000364600.1//...*1 | Exact | 26 | ggcugguccgaugguaguggguuauc |
| 10 | miRNA | mir-451a | Mature 5' | 22 | aaaccguuaccauuacugaguu |
| 11 | miRNA | mir-185 | Mature 5' | 22 | uggagagaaaggcaguuccuga |
| 12 | precursor | mir-451a | precursor miRNA | 16 | aaaccguuaccauuac |
| 13 | isomiR | mir-17 | Mature 5' sub | 22 | caaagugcuuacagugcaggua |
| 14 | MiscRNA | ENST00000364600.1//...*1 | Exact | 27 | ggcugguccgaugguaguggguuauca |
| 15 | precursor | mir-16-1//mir-16-2 | precursor miRNA | 19 | uagcagcacguaaauauug |
| 16 | isomiR | mir-30d | Mature 5' super | 24 | uguaaacauccccgacuggaagcu |
| 17 | isomiR | mir-185 | Mature 5' sub | 21 | uggagagaaaggcaguuccug |
| 18 | isomiR | mir-93 | Mature 5' sub | 21 | caaagugcuguucgugcaggu |
| 19 | isomiR | mir-101-1//mir-101-2 | Mature 3'super | 22 | uacaguacugugauaacugaag |
| 20 | isomiR | mir-30d | Mature 5' super | 23 | uguaaacauccccgacuggaagc |
| 21 | isomiR | mir-30d | Mature 5' sub | 21 | uguaaacauccccgacuggaa |
| 22 | isomiR | mir-145 | Mature 5' sub | 22 | guccaguuuucccaggaauccc |
| 23 | precursor | mir-15b | precursor miRNA | 19 | cgaaucauuauuugcugcu |
| 24 | precursor | mir-24-1//mir-24-2 | precursor miRNA | 19 | uggcucaguucagcaggaa |
| 25 | isomiR | mir-484 | Mature 5' sub | 21 | ucaggcucaguccccucccga |
| 26 | precursor | mir-191 | precursor miRNA | 20 | caacggaaucccaaaagcag |
| 27 | precursor | mir-221 | precursor miRNA | 20 | agcuacauugucugcugggu |
| 28 | isomiR | mir-29c | Mature 3' sub | 20 | uagcaccauuugaaaucggu |
| 29 | miRNA | let-7f-1//let-7f-2 | Mature 5' sub | 21 | ugagguaauagauuguauagu |

**Table 1-2**

| SEQ ID NO: | Class | Archetype | Type | Length (nucleotides) | Sequence |
|---|---|---|---|---|---|
| 30 | miRNA | mir-484 | Mature 5' | 22 | ucaggcucaguccccucccgau |
| 31 | miRNA | mir-130a | Mature 3' | 22 | cagugcaauguuaaaagggcau |
| 32 | isomiR | mir-191 | Mature 5' super | 24 | caacggaaucccaaaagcagcugu |
| 33 | isomiR | mir-486-1//mir-486-2 | Mature 5' sub | 20 | uccuguacugagcugccccg |
| 34 | isomiR | let-7d | Mature 3' sub | 20 | cuauacgaccugcugccuuu |
| 35 | precursor | mir-15b | precursor miRNA | 17 | uagcagcacaucauggu |
| 36 | precursor | mir-142 | precursor miRNA | 20 | cccauaaaguagaaagcacu |
| 37 | miRNA | let-7a-1//let-7a-2//let-7a-3 | Mature 5' | 22 | ugagguaguagguuguauaguu |
| 38 | miRNA | mir-151a | Mature 5' | 21 | ucgaggagcucacagucuagu |
| 39 | precursor | mir-103a-2//mir-103a-1//mir-107 | precursor miRNA | 19 | agcagcauuguacagggcu |
| 40 | miRNA | let-7f-1//let-7f-2 | Mature 5' | 22 | ugagguaguagauuguauaguu |
| 41 | miRNA | mir-148a | Mature 3' | 22 | ucagugcacuacagaacuuugu |
| 42 | precursor | mir-142 | precursor miRNA | 19 | cccauaaaguagaaagcac |
| 43 | isomiR | mir-26b | Mature 5' super | 22 | uucaaguaauucaggauagguu |
| 44 | isomiR | mir-197 | Mature 3' sub | 21 | uucaccaccuucuccacccag |
| 45 | precursor | mir-144 | precursor miRNA | 16 | uacaguauagaugaug |
| 46 | isomiR | mir-191 | Mature 5' sub | 22 | caacggaaucccaaaagcagcu |
| 47 | isomiR | mir-27a | Mature 3' sub | 20 | uucacaguggcuaaguuccg |
| 48 | isomiR | mir-3615 | Mature 3' super | 22 | ucucucggcuccucgcggcucg |
| 49 | miRNA | mir-423 | Mature 3' | 23 | agcucggucugaggccccucagu |
| 50 | isomiR | mir-223 | Mature 3' super | 23 | ugucaguuugucaaauaccccaa |
| 51 | isomiR | mir-223 | Mature 3' sub/super | 22 | gucaguuugucaaauaccccaa |
| 52 | isomiR | let-7a-1//let-7a-2//let-7a-3 | Mature 5' sub | 21 | ugagguaguagguuguauagu |
| 53 | isomiR | mir-191 | Mature 5' sub | 22 | aacggaaucccaaaagcagcug |
| 54 | miRNA | mir-15b | Mature 5' | 22 | uagcagcacaucaugguuuaca |
| 55 | miRNA | mir-223 | Mature 3' | 22 | uguraauuugucaaauacccca |
| 56 | precursor | mir-223 | precursor miRNA | 15 | ugucaguuugucaaa |
| 57 | isomiR | mir-223 | Mature 3' sub | 21 | gucaguuugucaaauacccca |
| 58 | isomiR | mir-144 | Mature 5' super | 23 | ggauaucaucauauacuguaagu |

**Table 1-3**

| SEQ ID NO: | Class | Archetype | Type | Length (nucleotides) | Sequence |
|---|---|---|---|---|---|
| 59 | isomiR | mir-223 | Mature 3' sub | 21 | ugucaguuugucaaauacccc |
| 60 | miRNA | mir-197 | Mature 3' | 22 | uucaccaccuucuccacccagc |
| 61 | miRNA | mir-144 | Mature 5' | 22 | ggauaucaucauauacuguaag |
| 62 | isomiR | mir-223 | Mature 3' sub | 20 | ugucaguuugucaaauaccc |
| 63 | isomiR | mir-23a | Mature 3' sub | 19 | aucacauugccagggauuu |
| 64 | precursor | mir-223 | precursor miRNA | 19 | ugucaguuugucaaauacc |
| 65 | isomiR | mir-4286 | Mature 5' super | 18 | accccacuccugguacca |
| 66 | miRNA | mir-769 | Mature 3' sub | 20 | ugggaucuccggggucuugg |
| 67 | LincRNA | ENST00000517335.1//...*3 | Exact | 31 | ccauguuggucaggcuggucuugaacuccug |
| 68 | ncRNA | ENST00000437898.1 | Exact | 15 | gagggaacgugagcu |
| 69 | miRNA | let-7g | Mature 5' | 22 | ugagguaguaguuuguacaguu |
| 70 | miRNA | mir-18a | Mature 5' | 23 | uaaggugcaucuagugcagauag |
| 71 | miRNA | mir-106b | Mature 5' | 21 | uaaagugcugacagugcagau |
| 72 | miRNA | mir-19b-1//mir-19b-2 | Mature 3' | 23 | ugugcaaauccaugcaaaacuga |
| 73 | isomiR | mir-876 | Mature 5' | 22 | uggauuucuuugugaaucacca |
| 74 | isomiR | mir-223 | Mature 3' sub | 17 | guuugucaaauacccca |
| 75 | miRNA | mir-425 | Mature 5' | 23 | aaugacacgaucacucccguuga |
| 76 | LincRNA | ENST00000626826.1 | Exact | 18 | aggaggaggaggaggacg |
| 77 | tRF | Homo_sapiens_tRNA-iMet-CAT-1-1//... | Exact | 20 | agaguggcgcagcggaagcg |
| 78 | miRNA | mir-22 | Mature 3' | 22 | aagcugccaguugaagaacugu |
| 79 | MiscRNA | ENST00000410769.1 | Exact | 15 | cacaaccaguuacca |
| 80 | ncRNA | ENST00000635274.1//...*5 | Exact | 17 | ggcuguagugcgcuaug |
| 81 | LincRNA | ENST00000567317.5 | Exact | 35 | gccugaggucuacugcugccuuauccagagcugcc |
| 82 | isomiR | mir-361 | Mature 3' sub | 20 | ucccccaggugugauucuga |
| 83 | LincRNA | ENST00000556266.1//...*4 | Exact | 16 | cuuaugcaggaggacc |
| 84 | isomiR | mir-320a | Mature 3' | 22 | aaaagcuggguugagagggcga |
| 85 | LincRNA | ENST00000607746.1 | Exact | 15 | agagcagaagggaag |

**Table 1-4**

| |
|---|
| *1:ENST00000364600.1//ENST00000577883.2//ENST00000577984.2//ENST00000516678.1//ENST00000516507.1//ENST00000481041.3//ENST0000057962 5.2//ENST00000365571.2//ENST00000578877.2//ENST00000364908.1 |
| *2:Homo_sapiens_tRNA-iMet-CAT-1-1//Homo_sapiens_tRNA-iMet-CAT-1-2//Homo_sapiens_tRNA-iMet-CAT-1-3//Homo_sapiens_tRNA-iMct-CAT-1-4//Hom o_sapiens_tRNA-iMel-CAT-1-5//Homo_sapiens_tRNA-iMet-CAT-1 -6//Homo_sapiens_tRNA-iMet-CAT-1-7//Homo_sapiens_tRNA-iMet-CAT-1-8//Homo_sapie ns_tRNA-iMet-CAT-2-1 |
| *3:ENST00000517335.1//ENST00000499583.1//ENST00000455531.1//ENST00000398461.5//ENST00000612531.1//ENST00000425800.1//ENST0000045525 3.6//ENST00000454128.2//ENST00000602737.5//ENST00000414209.5//ENST00000452320.3//ENST00000640355.1//ENST00000638174.1//ENST000005813 98.1 |
| *4:ENST00000556266.1//ENST00000554441.5//ENST00000557532.5//ENST00000554694.1 |
| *5:ENST00000635274.1//ENST00000461926.3//ENST00000582522.2//ENST00000469617.3//ENST00000476501.3//ENST00000581392.2//ENST0000048730 9.3//ENST00000481857.3//ENST00000486780.3//ENST00000463926.3//ENST00000478498.3//ENST00000577207.2//ENST00000463397.3//ENST000006193 03.1//ENST00000470786.3//ENST00000493013.3//ENST00000618786.1//ENST00000581458.2//ENST00000491451.3//ENST00000467883.3//ENST00000479 428.3//ENST00000496780.3//ENST00000585237.2//ENST00000610674.1//ENST00000490232.3//ENST00000584058.2 |

Most of those miRNAs or the like show the logarithm of the ratio of the abundance in serum from patients with Alzheimer's disease to the abundance in serum from healthy subjects (represented by "log FC," which means the logarithm of FC (fold change) to base 2) is more than 1.0 in absolute value (that is, a ratio of not less than 2 or not more than 1/2) as indicated in Table 2 below, which is statistically significant (*t*-test: *p* < 0.05).

The abundance of miRNAs or the like represented by SEQ ID NOs: 1 to 22 and 66 to 71 is higher in patients with Alzheimer's disease than in healthy subjects, while the abundance of miRNAs or the like represented by SEQ ID NOs: 23 to 65 and 72 to 85 is lower in patients with Alzheimer's disease than in healthy subjects.

Among those, the miRNAs or the like represented by SEQ ID NOs: 1, 3, 11, 12, 2, 4 to 10, 46 to 65, 84, and 85 have a log FC value of not less than 1.5 in absolute value and thus function as indexes with especially high sensitivity, and are preferable.

The accuracy of each biomarker is indicated using the area under the ROC curve (AUC: Area Under Curve) as an index, and biomarkers with an AUC value of 0.7 or higher are generally considered effective. AUC values of 0.90 or higher, 0.97 or higher. 0.98 or higher, and 1.00 correspond to biomarkers with high accuracy, very high accuracy, even higher accuracy, and complete accuracy (with no false-positive and false-negative events), respectively. Thus, the AUC value of each biomarker is likewise preferably 0.90, more preferably not less than 0.97, still more preferably not less than 0.98, and most preferably 0.99 in the present invention. The miRNAs or the like represented by SEQ ID NOs: 1, 3, 11, and 12 are preferable because of an AUC value of 0.97 or higher; among those, the miRNAs or the like represented by SEQ ID NOs: 1 and 3 are more preferable because of an AUC value of 0.98 or higher; the miRNA represented by SEQ ID NO: 1 is most preferable because of an AUC value of 1.00.

The test sample is not specifically limited, provided that the test sample is a body fluid containing miRNAs; typically, it is preferable to use a blood sample (including plasma, serum, and whole blood). It is simple and preferable to use serum or plasma as a test sample.

The abundance of each miRNA or the like is preferably measured (quantified) using a next-generation sequencer. Any instrument may be used and is not limited to a specific type of instrument, provided that the instrument determines sequences, similarly to next-generation sequencers. In the method of the present invention, as specifically described in Examples below, use of a next-generation sequencer is preferred over quantitative reverse-transcription PCR (qRT-PCR) which is widely used for quantification of miRNAs, to perform measurements from the viewpoint of accuracy because miRNAs or the like to be quantified include, for example, isomiRs, in which only one or more nucleotides are deleted from or added to the 5' and/or 3' ends of the original mature miRNAs thereof, and which should be distinguished from the original miRNAs when measured. Briefly, though details will be described specifically in Examples below, the quantification method can be performed, for example, as follows. When the RNA content in serum or plasma is constant, among reads measured in a next-generation sequencing analysis of the RNA content, the number of reads for each isomiR or mature miRNA per million reads is considered as the measurement value, where the total counts of reads with human-derived sequences are normalized to one million reads. When the RNA content in serum or plasma is variable in comparison with healthy subjects due to a disease, miRNAs showing little abundance variation in serum and plasma may be used. In cases where the abundance of miRNAs or the like in serum or plasma is measured, at least one miRNA selected from the group consisting of let-7g-5p, miR-425-3p, and miR-425-5p is preferably used as an internal control, which are miRNAs showing little abundance variation in serum and plasma.

The cut-off value for the abundance of each miRNA or the like for use in evaluation is preferably determined based on the presence or absence of a statistically significant difference (*t*-test; *p <* 0.05, preferably *p* < 0.01, more preferably *p* < 0.001) from healthy subjects with regard to the abundance of the miRNA or the like. Specifically, the value of log₂ read counts (the cut-off value) can be preferably determined for each miRNA or the like, for example, at which the false-positive rate is optimal (the lowest); for example, the cut-off values (the values of log₂ read counts) for several miRNAs or the like are as indicated in Table 2. The cut-off values indicated in Table 2 are only examples, and other values may be employed as cut-off values as long as those values are appropriate to determine statistically significant difference. Additionally, the optimal cut-off values vary among different populations of patients and healthy subjects from which data is collected. However, a cut-off value may be set such that the cut-off value is within the range of, usually ±20%, particularly ±10%, from the cut-off value indicated in Table 2.

Additionally, the abundance of a miRNA and that of each isomiR thereof are different between patients and healthy subjects, even among miRNAs or the like derived from the same archetype. Thus, the measurement of a certain miRNA and an isomiR thereof in one patient, which are derived from the same archetype, can assist in Alzheimer's disease detection based on the abundance ratio thereof. Because small differences in nucleotide sequence should be accurately distinguished, when the abundance of a certain miRNA and that of an isomiR thereof are measured, use of a next-generation sequencer is preferred over quantitative reverse-transcription PCR (qRT-PCR) which is typically used in miRNA measurement, to perform measurements.

Each of the above miRNAs or the like is statistically significantly different in abundance between patients with Alzheimer's disease and healthy subjects, and may thus be used alone as an index. However, a combination of multiple miRNAs or the like may also be used as an index, which can assist in more accurate detection of Alzheimer's disease.

Moreover, a method of detecting the abundance of miRNAs or the like in a test sample from an individual suspected of having or affected with Alzheimer's disease is also provided. That is, a method of detecting the abundance of at least one of miRNAs, isomiRs, precursor miRNAs, ncRNAs, transfer RNA fragments, LincRNAs, or MiscRNAs whose nucleotide sequence is represented by any one of SEQ ID NOs: 1, 3, 11, 12, 2, 4 to 10, and 13 to 85 in a test sample from an individual suspected of having or affected with Alzheimer's disease is also provided, wherein the method includes the steps of:
collecting a blood sample from the individual; and
measuring the abundance of the miRNA(s), isomiR(s). precursor miRNA(s), ncRNA(s), transfer RNA fragment(s), LincRNA(s). or MiscRNA(s) in the blood sample by means of a next-generation sequencer or qRT-PCR;
wherein the abundance of at least one of the miRNAs, isomiRs, precursor miRNAs, ncRNAs, transfer RNA fragments, LincRNAs, or MiscRNAs whose nucleotide sequence is represented by any one of SEQ ID NOs: 1 to 22 and 66 to 71 is higher in patients than in healthy subjects, or the abundance of at least one of the miRNAs, isomiRs, precursor miRNAs, ncRNAs, transfer RNA fragments, LincRNAs, or MiscRNAs whose nucleotide sequence is represented by any one of SEQ ID NOs: 23 to 65 and 72 to 85 is lower in patients than in healthy subjects.

Additionally, in cases where the detection of Alzheimer's disease is successfully achieved by the above-described method of the present invention, an effective amount of an Alzheimer's disease drug can be administered to patients in whom Alzheimer's disease is detected, to treat the Alzheimer's disease. Examples of the Alzheimer's disease drug can include donepezil, rivastigmine, galantamine, and memantine.

The present invention will be specifically described below by way of examples and comparative examples. However, the present invention is not limited to the examples below.

### Examples 1 to 85

### 1. Materials and Methods

### (1) Clinical Samples

Plasma samples from 43 patients with Alzheimer's disease and from 32 healthy subjects were used.

### (2) Extraction of RNA in Serum

Extraction of RNA in serum was performed using the miRNeasy Mini kit (QIAGEN).
1) Each frozen serum sample was thawed and centrifuged at 10,000 rpm for 5 minutes at room temperature to precipitate aggregated proteins and blood cell components.
2) To a new 1.5-mL tube, 200 µL of the supernatant was transferred.
3) To the tube, 1000 µL of the QIAzol Lysis Reagent was added and mixed thoroughly to denature protein components.
4) To the tube, 10 µL of 0.05 nM cel-miR-39 was added as a control RNA for RNA extraction, mixed by pipetting, and then left to stand at room temperature for 5 minutes.
5) To promote separation of the aqueous and organic solvent layers, 200 µL of chloroform was added to the tube, mixed thoroughly, and left to stand at room temperature for 3 minutes.
6) The tube was centrifuged at 12,000 x g for 15 minutes at 4°C and 650 µL of the upper aqueous layer was transferred to a new 2-mL tube.
7) For the separation of RNA, 975 µL of 100% ethanol was added to the tube and mixed by pipetting.
8) To a miRNeasy Mini spin column (hereinafter referred to as "column"), 650 µL of the mixture in the step 7 was transferred, left to stand at room temperature for 1 minute, and then centrifuged at 8000 x g for 15 seconds at room temperature to allow RNA to be adsorbed on the filter of the column. The flow-through solution from the column was discarded.
9) The step 8 was repeated until the total volume of the solution of the step 7 was filtered through the column to allow all the RNA to be adsorbed on the filter.
10) To remove impurities attached on the filter, 650 µL of Buffer RWT was added to the column and centrifuged at 8000 x g for 15 seconds at room temperature. The flow-through solution from the column was discarded.
11) To clean the RNA adsorbed on the filter, 500 µL of Buffer RPE was added to the column and centrifuged at 8000 x g for 15 seconds at room temperature. The flow-through solution from the column was discarded.
12) To clean the RNA adsorbed on the filter, 500 µL of Buffer RPE was added to the column and centrifuged at 8000 x g for 2 minutes at room temperature. The flow-through solution from the column was discarded.
13) To completely remove any solution attached on the filter, the column was placed in a new 2-mL collection tube and centrifuged at 10,000 x g for 1 minute at room temperature.
14) The column was placed into a 1.5-mL tube and 50 µL of RNase-free water was added thereto and left to stand at room temperature for 1 minute.
15) Centrifugation was performed at 8000 x g for 1 minute at room temperature to elute the RNA adsorbed on the filter. The eluted RNA was used in the following experiment without further purification and the remaining portion of the eluted RNA was stored at -80°C.

### (4) Quantification of miRNAs or the Like

The quantification of miRNAs or the like was performed as follows. In cases where miRNAs or the like from, for example, two groups were quantified, extracellular vesicles (including exosomes) isolated by the same method were used to extract RNAs through the same method, from which cDNA libraries were prepared and then analyzed by next-generation sequencing. The next-generation sequencing analysis is not limited by a particular instrument, provided that the instrument determines sequences.

### 2. Results

The results are presented in Table 2.

**Table 2-1**

| Example | SEQ ID NO: | Class | Archetype | Type | Length (nucleotides) | Average in healthy subjects | Average in AD patients* | log FC | AUC | Cut-off value |
|---|---|---|---|---|---|---|---|---|---|---|
| Example 1 | 1 | miRNA | mir-340 | Mature 3' | 22 | 1 | 2984 | 11.1 | 1.000 | 8.90 |
| Example 2 | 2 | miRNA | mir-122 | Mature 5' | 22 | 216 | 1611 | 2.9 | 0.852 | 8.98 |
| Example 3 | 3 | isomiR | mir-181b-1//mir-181b-2 | Mature 5' sub | 22 | 96 | 695 | 2.9 | 0.993 | 7.60 |
| Example 4 | 4 | isomiR | mir-451a | Mature 5' super | 24 | 568 | 3471 | 2.6 | 0.919 | 9.77 |
| Example 5 | 5 | isomiR | mir-20a | Mature 5' sub | 22 | 173 | 798 | 2.2 | 0.83 | 8.36 |
| Example 6 | 6 | miRNA | mir-20a | Mature 5' | 23 | 744 | 3149 | 2.1 | 0.881 | 10.63 |
| Example 7 | 7 | isomiR | mir-20a | Mature 5' sub | 21 | 519 | 1822 | 1.8 | 0.652 | 10.70 |
| Example 8 | 8 | isomiR | mir-451a | Mature 5' super | 23 | 20679 | 72278 | 1.8 | 0.926 | 14.48 |
| Example 9 | 9 | MiscRNA | ENST00000364600.1//...*1 | Exact | 26 | 1870 | 5975 | 1.7 | 0.941 | 11.81 |
| Example 10 | 10 | miRNA | mir-451 a | Mature 5' | 22 | 49304 | 148434 | 1.6 | 0.852 | 16.84 |
| Example 11 | 11 | miRNA | mir-185 | Mature 5' | 22 | 2520 | 7180 | 1.5 | 0.97 | 11.94 |
| Example 12 | 12 | precursor | mir-451 a | precursor miRNA | 16 | 252 | 645 | 1.4 | 0.97 | 9.14 |
| Example 13 | 13 | isomiR | mir-17 | Mature 5' sub | 22 | 284 | 726 | 1.4 | 0.859 | 9.10 |
| Example 14 | 14 | MiscRNA | ENST00000364600.1//...*1 | Exact | 27 | 206 | 492 | 1.3 | 0.822 | 8.46 |
| Example 15 | 15 | precursor | mir-16-1//mir-16-2 | precursor miRNA | 19 | 278 | 616 | 1.1 | 0.844 | 8.64 |
| Example 16 | 16 | isomiR | mir-30d | Mature 5' super | 24 | 444 | 941 | 1.1 | 0.837 | 9.10 |
| Example 17 | 17 | isomiR | mir-185 | Mature 5' sub | 21 | 2331 | 4824 | 1 | 0.919 | 11.78 |
| Example 18 | 18 | isomiR | mir-93 | Mature 5' sub | 21 | 433 | 891 | 1 | 0.815 | 10.00 |

**Table 2-2**

| Example | SEQ ID NO: | Class | Archetype | Type | Length (nucleotides) | Average in healthy subjects | Average in AD patients* | log FC | AUC | Cut-off value |
|---|---|---|---|---|---|---|---|---|---|---|
| Example 19 | 19 | isomiR | mir-101-1//mir-101-2 | Mature 3' super | 22 | 941 | 1915 | 1 | 0.667 | 11.04 |
| Example 20 | 20 | isomiR | mir-30d | Mature 5' super | 23 | 182 | 367 | 1 | 0.785 | 8.00 |
| Example 21 | 21 | isomiR | mir-30d | Mature 5' sub | 21 | 661 | 1310 | 1 | 0.726 | 9.89 |
| Example 22 | 22 | isomiR | mir-145 | Mature 5' sub | 22 | 381 | 745 | 1 | 0.756 | 9.53 |
| Example 23 | 23 | precursor | mir-15b | precursor miRNA | 19 | 444 | 230 | -1 | 0.844 | 7.40 |
| Example 24 | 24 | precursor | mir-24-1//mir-24-2 | precursor miRNA | 19 | 607 | 306 | -1 | 0.748 | 8.06 |
| Example 25 | 25 | isomiR | mir-484 | Mature 5' sub | 21 | 6274 | 3127 | -1 | 0.933 | 12.30 |
| Example 26 | 26 | precursor | mir-191 | precursor miRNA | 20 | 3638 | 1794 | -1 | 0.852 | 11.37 |
| Example 27 | 27 | precursor | mir-221 | precursor miRNA | 20 | 561 | 274 | -1 | 0.741 | 8.44 |
| Example 28 | 28 | isomiR | mir-29c | Mature 3' sub | 20 | 351 | 170 | -1 | 0.926 | 7.53 |
| Example 29 | 29 | miRNA | let-7f.1//let-7f-2 | Mature 5' sub | 21 | 866 | 414 | -1.1 | 0.83 | 9.10 |
| Example 30 | 30 | miRNA | mir-484 | Mature 5' | 22 | 2241 | 1055 | -1.1 | 0.941 | 10.60 |
| Example 31 | 31 | miRNA | mir-130a | Mature 3' | 22 | 473 | 221 | -1.1 | 0.844 | 8.39 |
| Example 32 | 32 | isomiR | mir-191 | Mature 5' super | 24 | 475 | 217 | -1.1 | 0.711 | 7.54 |
| Example 33 | 33 | isomiR | mir-486-1//mir-486-2 | Mature 5' sub | 20 | 6476 | 2942 | -1.1 | 0.948 | 12.18 |
| Example 34 | 34 | isomiR | let-7d | Mature 3' sub | 20 | 168 | 76 | -1.1 | 0.859 | 7.14 |

**Table 2-3**

| Example | SEQ ID NO: | Class | Archetype | Type | Length (nucleotides) | Average in healthy subjects | Average in AD patients* | log FC | AUC | Cut-off value |
|---|---|---|---|---|---|---|---|---|---|---|
| Example 35 | 35 | precursor | mir-15b | precursor miRNA | 17 | 187 | 81 | -1.2 | 0.696 | 6.15 |
| Example 36 | 36 | precursor | mir-142 | precursor miRNA | 20 | 2105 | 906 | -1.2 | 0.956 | 10.29 |
| Example 37 | 37 | miRNA | let-7a-1//let-7a-2//let-7a-3 | Mature 5' | 22 | 1853 | 795 | -1.2 | 0.807 | 9.63 |
| Example 38 | 38 | miRNA | mir-151a | Mature 5' | 21 | 564 | 233 | -1.3 | 0.859 | 8.42 |
| Example 39 | 39 | precursor | mir-103a-2/7mir-103a-1//mir -107 | precursor miRNA | 19 | 3502 | 1437 | -1.3 | 0.867 | 11.03 |
| Example 40 | 40 | miRNA | let-7f-1//let-7f-2 | Mature 5' | 22 | 481 | 196 | -1.3 | 0.844 | 7.71 |
| Example 41 | 41 | miRNA | mir-148a | Mature 3' | 22 | 346 | 135 | -1.4 | 0.793 | 7.99 |
| Example 42 | 42 | precursor | mir-142 | precursor miRNA | 19 | 294 | 114 | -1.4 | 0.889 | 6.77 |
| Example 43 | 43 | isomiR | mir-26b | Mature 5' super | 22 | 177 | 68 | -1.4 | 0.815 | 5.20 |
| Example 44 | 44 | isomiR | mir-197 | Mature 3' sub | 21 | 398 | 147 | -1.4 | 0.919 | 7.54 |
| Example 45 | 45 | precursor | mir-144 | precursor miRNA | 16 | 333 | 122 | -14 | 0.748 | 6.14 |
| Example 46 | 46 | isomiR | mir-191 | Mature 5' sub | 22 | 1956 | 700 | -1.5 | 0.933 | 10.36 |
| Example 47 | 47 | isomiR | mir-27a | Mature 3' sub | 20 | 3273 | 1150 | -1.5 | 0.859 | 11.30 |
| Example 48 | 48 | isomiR | mir-3615 | Mature 3' super | 22 | 128 | 45 | -1.5 | 0.793 | 5.46 |
| Example 49 | 49 | miRNA | mir-423 | Mature 3' | 23 | 295 | 101 | -1.6 | 0.904 | 7.36 |
| Example 50 | 50 | isomiR | mir-223 | Mature 3' super | 23 | 42236 | 13998 | -1.6 | 0.83 | 14.44 |

**Table 2-4**

| Example | SEQ ID NO: | Class | Archetype | Type | Length (nucleotides) | Average in healthy subjects | Average in AD patients* | log FC | AUC | Cut-off value |
|---|---|---|---|---|---|---|---|---|---|---|
| Example 51 | 51 | isomiR | mir-223 | Mature 3' sub/super | 22 | 12615 | 4139 | -1.6 | 0.859 | 13.47 |
| Example 52 | 52 | isomiR | let-7a-1//let-7a-2//let-7a-3 | Mature 5' sub | 21 | 2213 | 675 | -1.7 | 0.904 | 10.30 |
| Example 53 | 53 | isomiR | mir-191 | Mature 5' sub | 22 | 713 | 208 | -1.8 | 0.948 | 8.57 |
| Example 54 | 54 | miRNA | mir-15b | Mature 5' | 22 | 747 | 213 | -1.8 | 0.896 | 8.86 |
| Example 55 | 55 | miRNA | mir-223 | Mature 3' | 22 | 39732 | 10170 | -2 | 0.837 | 13.45 |
| Example 56 | 56 | precursor | mir-223 | precursor miRNA | 15 | 108 | 27 | -2 | 0.822 | 5.83 |
| Example 57 | 57 | isomiR | mir-223 | Mature 3' sub | 21 | 7627 | 1711 | -2.2 | 0.844 | 12.22 |
| Example 58 | 58 | isomiR | mir-144 | Mature 5' super | 23 | 309 | 66 | -2.2 | 0.867 | 7.00 |
| Example 59 | 59 | isomiR | mir-223 | Mature 3' sub | 21 | 5364 | 985 | -2.4 | 0.889 | 10.15 |
| Example 60 | 60 | miRNA | mir-197 | Mature 3' | 22 | 877 | 147 | -2.6 | 0.963 | 8.54 |
| Example 61 | 61 | miRNA | mir-144 | Mature 5' | 22 | 243 | 36 | -2.7 | 0.911 | 6.67 |
| Example 62 | 62 | isomiR | mir-223 | Mature 3' sub | 20 | 3274 | 430 | -2.9 | 0.867 | 8.88 |
| Example 63 | 63 | isomiR | mir-23a | Mature 3' sub | 19 | 496 | 57 | -3.1 | 0.896 | 6.64 |
| Example 64 | 64 | precursor | mir-223 | precursor miRNA | 19 | 1849 | 209 | -3.1 | 0.904 | 8.86 |
| Example 65 | 65 | isomiR | mir-4286 | Mature 5' super | 18 | 515 | 42 | -3.6 | 0.896 | 6.98 |
| Example 66 | 66 | isomiR | mir-769 | Mature 3' sub | 20 | 3789 | 9843 | 1.38 | 0.81 | 12.5 |
| Example 67 | 67 | LincRNA | ENST00000517335.1//...*3 | Exact | 31 | 1589 | 3580 | 1.17 | 0.759 | 11.2 |

**Table 2-5**

| Example | SEQ ID NO: | Class | Archetype | Type | Length (nucleotides) | Average in healthy subjects | Average in AD patients* | log FC | AUC | Cut-off value |
|---|---|---|---|---|---|---|---|---|---|---|
| Example 68 | 68 | ncRNA | ENST00000437898.1 | Exact | 15 | 25718 | 57524 | 1.16 | 0.783 | 15.13 |
| Example 69 | 69 | miRNA | let-7g | Mature 5' | 22 | 4370 | 7081 | 0.70 | 0.684 | 12.09 |
| Example 70 | 70 | miRNA | mir-18a | Mature 5' | 23 | 1811 | 2713 | 0.58 | 0.619 | 11.37 |
| Example 71 | 71 | miRNA | mir-106b | Mature 5' | 21 | 2501 | 3470 | 0.47 | 0.587 | 11.53 |
| Example 72 | 72 | miRNA | mir-19b-1//mir-19b-2 | Mature 3' | 23 | 97040 | 73555 | -0.40 | 0.695 | 16.69 |
| Example 73 | 73 | miRNA | mir-876 | Mature 5' | 22 | 5753 | 3772 | -0.61 | 0.762 | 12.24 |
| Example 74 | 74 | isomiR | mir-223 | Mature 3' sub | 17 | 2889 | 1880 | -0.62 | 0.672 | 11.76 |
| Example 75 | 75 | miRNA | mir-425 | Mature 5' | 23 | 6399 | 4114 | -0.64 | 0.689 | 12.46 |
| Example 76 | 76 | LincRNA | ENST00000626826.1 | Exact | 18 | 3513 | 2080 | -0.76 | 0.738 | 11.3 |
| Example 77 | 77 | tRF | Homo_sapiens_tRNA-iMet-CAT-1-1//...*2 | Exact | 20 | 1446 | 768 | -0.91 | 0.779 | 10.4 |
| Example 78 | 78 | miRNA | mir-22 | Mature 3' | 22 | 6348 | 3301 | -0.94 | 0.677 | 12.22 |
| Example 79 | 79 | MiscRNA | ENST00000410769.1 | Exact | 15 | 3136 | 1488 | -1.08 | 0.83 | 10.9 |
| Example 80 | 80 | ncRNA | ENST00000635274.1//...*5 | Exact | 17 | 1674 | 739 | -1.18 | 0.799 | 9.81 |
| Example 81 | 81 | LincRNA | ENST00000567317.5 | Exact | 35 | 1386 | 593 | -1.22 | 0.803 | 9.96 |
| Example 82 | 82 | isomiR | mir-361 | Mature 3' sub | 20 | 1714 | 677 | -1.34 | 0.84 | 9.92 |
| Example 83 | 83 | LincRNA | ENST00000556266.1//...*4 | Exact | 16 | 1237 | 481 | -1.36 | 0.893 | 9.29 |
| Example 84 | 84 | miRNA | mir-320a | Mature 3' | 22 | 24669 | 7832 | -1.66 | 0.78 | 13.76 |
| Example 85 | 85 | LincRNA | ENST00000607746.1 | Exact | 15 | 1115 | 258 | -2.11 | 0.92 | 8.99 |

| | | | | | | | | | | |
|---|---|---|---|---|---|---|---|---|---|---|
| *AD: Alzheimer's disease *1 to *5 in this table represent the same molecules represented by *1 to *5 in Table 1. | | | | | | | | | | |

As seen in these results, among those sequences, the abundance of the miRNAs or the like represented by SEQ ID NOs: 1 to 22 and 66 to 71 was significantly higher in the patients with Alzheimer's disease than in the healthy subjects, and the abundance of the miRNAs or the like represented by SEQ ID NOs: 23 to 65 and 72 to 85 was significantly lower in the patients with Alzheimer's disease than in the healthy subjects. Moreover, all the *p*-values determined by t-test in Examples 1 to 85 were less than 0.05, indicating the effectiveness in detection of Alzheimer's disease.

## Claims

1. A method of assisting the detection of Alzheimer's disease, using as an index the abundance of at least one of miRNAs, isomiRs, precursor miRNAs, ncRNAs, transfer RNA fragments, LincRNAs, or MiscRNAs contained in a test sample isolated from a living body, whose nucleotide sequence is represented by any one of SEQ ID NOs: 1, 3, 12, 2, 4 to 11, and 13 to 85, wherein a higher abundance of at least one of the miRNAs, isomiRs, precursor miRNAs, ncRNAs, transfer RNA fragments, LincRNAs, or MiscRNAs whose nucleotide sequence is represented by any one of SEQ ID NOs: 1 to 22 and 66 to 71 than that of healthy subjects or a lower abundance of at least one of the miRNAs, isomiRs, precursor miRNAs, ncRNAs, transfer RNA fragments, LincRNAs, or MiscRNAs whose nucleotide sequence is represented by any one of SEQ ID NOs: 23 to 65 and 72 to 85 than that of healthy subjects indicates a higher likelihood of having Alzheimer's disease.

2. The method according to claim 1, wherein the abundance of at least one of miRNAs, isomiRs, precursor miRNAs, ncRNAs, transfer RNA fragments, LincRNAs, or MiscRNAs whose nucleotide sequence is represented by any one of SEQ ID NOs: 1, 3, 12, 2, 4 to 11, and 13 to 65 is used as an index.

3. The method according to claim 1, wherein the abundance of at least one of miRNAs, isomiRs, precursor miRNAs, transfer RNA fragments, LincRNAs, or MiscRNAs whose nucleotide sequence is represented by any one of SEQ ID NOs: 1, 3, 12, 2, 4 to 11, 46 to 65, 84, and 85 is used as an index.

4. The method according to claim 2, wherein the abundance of at least one of miRNAs, isomiRs, precursor miRNAs, transfer RNA fragments, or MiscRNAs whose nucleotide sequence is represented by any one of SEQ ID NOs: 1, 3, 12, 2, 4 to 11, and 46 to 65 is used as an index.

5. The method according to claim 4, wherein the abundance of at least one of miRNAs, isomiRs, or precursor miRNAs whose nucleotide sequence is represented by any one of SEQ ID NOs: 1, 3, 12, and 11 is used as an index.


SEQUENCE LISTING

<110>  National University Corporation Hiroshima University

<120>  Method of aiding detection of Alzheimer's disease

<130>  PF630-PCT

<160>  85

<170>  PatentIn version 3.5

<210>  1
<211>  22
<212>  RNA
<213>  Homo sapiens

<400>  1
uccgucucag uuacuuuaua gc                                                22

<210>  2
<211>  22
<212>  RNA
<213>  Homo sapiens

<400>  2
uggaguguga caaugguguu ug                                                22

<210>  3
<211>  22
<212>  RNA
<213>  Homo sapiens

<400>  3
acauucauug cugucggugg gu                                                22

<210>  4
<211>  24
<212>  RNA
<213>  Homo sapiens

<400>  4
aaaccguuac cauuacugag uuua                                              24

<210>  5
<211>  22
<212>  RNA
<213>  Homo sapiens

<400>  5
uaaagugcuu auagugcagg ua                                                22

<210>  6
<211>  23
<212>  RNA
<213>  Homo sapiens

<400>  6

uaaagugcuu auagugcagg uag                                              23


<210>  7
<211>  21
<212>  RNA
<213>  Homo sapiens

<400>  7
uaaagugcuu auagugcagg u                                                21


<210>  8
<211>  23
<212>  RNA
<213>  Homo sapiens

<400>  8
aaaccguuac cauuacugag uuu                                              23


<210>  9
<211>  26
<212>  RNA
<213>  Homo sapiens

<400>  9
ggcugguccg augguagugg guuauc                                           26


<210>  10
<211>  22
<212>  RNA
<213>  Homo sapiens

<400>  10
aaaccguuac cauuacugag uu                                               22


<210>  11
<211>  22
<212>  RNA
<213>  Homo sapiens

<400>  11
uggagagaaa ggcaguuccu ga                                               22


<210>  12
<211>  16
<212>  RNA
<213>  Homo sapiens

<400>  12
aaaccguuac cauuac                                                      16


<210>  13
<211>  22
<212>  RNA
<213>  Homo sapiens

<400>  13

caaagugcuu acagugcagg ua                                                22

<210>  14
<211>  27
<212>  RNA
<213>  Homo sapiens

<400>  14
ggcugguccg augguagugg guuauca                                           27

<210>  15
<211>  19
<212>  RNA
<213>  Homo sapiens

<400>  15
uagcagcacg uaaauauug                                                    19

<210>  16
<211>  24
<212>  RNA
<213>  Homo sapiens

<400>  16
uguaaacauc cccgacugga agcu                                              24

<210>  17
<211>  21
<212>  RNA
<213>  Homo sapiens

<400>  17
uggagagaaa ggcaguuccu g                                                 21

<210>  18
<211>  21
<212>  RNA
<213>  Homo sapiens

<400>  18
caaagugcug uucgugcagg u                                                 21

<210>  19
<211>  22
<212>  RNA
<213>  Homo sapiens

<400>  19
uacaguacug ugauaacuga ag                                                22

<210>  20
<211>  23
<212>  RNA
<213>  Homo sapiens

<400>  20

uguaaacauc cccgacugga agc                                              23

<210>  21
<211>  21
<212>  RNA
<213>  Homo sapiens

<400>  21
uguaaacauc cccgacugga a                                                21

<210>  22
<211>  22
<212>  RNA
<213>  Homo sapiens

<400>  22
guccaguuuu cccaggaauc cc                                               22

<210>  23
<211>  19
<212>  RNA
<213>  Homo sapiens

<400>  23
cgaaucauua uuugcugcu                                                   19

<210>  24
<211>  19
<212>  RNA
<213>  Homo sapiens

<400>  24
uggcucaguu cagcaggaa                                                   19

<210>  25
<211>  21
<212>  RNA
<213>  Homo sapiens

<400>  25
ucaggcucag uccccucccg a                                                21

<210>  26
<211>  20
<212>  RNA
<213>  Homo sapiens

<400>  26
caacggaauc ccaaaagcag                                                  20

<210>  27
<211>  20
<212>  RNA
<213>  Homo sapiens

<400>  27

agcuacauug ucugcugggu                                                    20

<210>  28
<211>  20
<212>  RNA
<213>  Homo sapiens

<400>  28
uagcaccauu ugaaaucggu                                                    20

<210>  29
<211>  21
<212>  RNA
<213>  Homo sapiens

<400>  29
ugagguagua gauuguauag u                                                  21

<210>  30
<211>  22
<212>  RNA
<213>  Homo sapiens

<400>  30
ucaggcucag uccccucccg au                                                 22

<210>  31
<211>  22
<212>  RNA
<213>  Homo sapiens

<400>  31
cagugcaaug uuaaaagggc au                                                 22

<210>  32
<211>  24
<212>  RNA
<213>  Homo sapiens

<400>  32
caacggaauc ccaaaagcag cugu                                               24

<210>  33
<211>  20
<212>  RNA
<213>  Homo sapiens

<400>  33
uccuguacug agcugccccg                                                    20

<210>  34
<211>  20
<212>  RNA
<213>  Homo sapiens

<400>  34

cuauacgacc ugcugccuuu                                                    20

<210>  35
<211>  17
<212>  RNA
<213>  Homo sapiens

<400>  35
uagcagcaca ucauggu                                                       17

<210>  36
<211>  20
<212>  RNA
<213>  Homo sapiens

<400>  36
cccauaaagu agaaagcacu                                                    20

<210>  37
<211>  22
<212>  RNA
<213>  Homo sapiens

<400>  37
ugagguagua gguuguauag uu                                                 22

<210>  38
<211>  21
<212>  RNA
<213>  Homo sapiens

<400>  38
ucgaggagcu cacagucuag u                                                  21

<210>  39
<211>  19
<212>  RNA
<213>  Homo sapiens

<400>  39
agcagcauug uacagggcu                                                     19

<210>  40
<211>  22
<212>  RNA
<213>  Homo sapiens

<400>  40
ugagguagua gauuguauag uu                                                 22

<210>  41
<211>  22
<212>  RNA
<213>  Homo sapiens

<400>  41

ucagugcacu acagaacuuu gu                                                22

<210>  42
<211>  19
<212>  RNA
<213>  Homo sapiens

<400>  42
cccauaaagu agaaagcac                                                    19

<210>  43
<211>  22
<212>  RNA
<213>  Homo sapiens

<400>  43
uucaaguaau ucaggauagg uu                                                22

<210>  44
<211>  21
<212>  RNA
<213>  Homo sapiens

<400>  44
uucaccaccu ucuccaccca g                                                 21

<210>  45
<211>  16
<212>  RNA
<213>  Homo sapiens

<400>  45
uacaguauag augaug                                                       16

<210>  46
<211>  22
<212>  RNA
<213>  Homo sapiens

<400>  46
caacggaauc ccaaaagcag cu                                                22

<210>  47
<211>  20
<212>  RNA
<213>  Homo sapiens

<400>  47
uucacagugg cuaaguuccg                                                   20

<210>  48
<211>  22
<212>  RNA
<213>  Homo sapiens

<400>  48

ucucucggcu ccucgcggcu cg                                                22

<210>  49
<211>  23
<212>  RNA
<213>  Homo sapiens

<400>  49
agcucggucu gaggccccuc agu                                               23

<210>  50
<211>  23
<212>  RNA
<213>  Homo sapiens

<400>  50
ugucaguuug ucaaauaccc caa                                               23

<210>  51
<211>  22
<212>  RNA
<213>  Homo sapiens

<400>  51
gucaguuugu caaauacccc aa                                                22

<210>  52
<211>  21
<212>  RNA
<213>  Homo sapiens

<400>  52
ugagguagua gguuguauag u                                                 21

<210>  53
<211>  22
<212>  RNA
<213>  Homo sapiens

<400>  53
aacggaaucc caaaagcagc ug                                                22

<210>  54
<211>  22
<212>  RNA
<213>  Homo sapiens

<400>  54
uagcagcaca ucaugguuua ca                                                22

<210>  55
<211>  22
<212>  RNA
<213>  Homo sapiens

<400>  55

ugucaguuug ucaaauaccc ca                                                22

<210>  56
<211>  15
<212>  RNA
<213>  Homo sapiens

<400>  56
ugucaguuug ucaaa                                                        15

<210>  57
<211>  21
<212>  RNA
<213>  Homo sapiens

<400>  57
gucaguuugu caaauacccc a                                                 21

<210>  58
<211>  23
<212>  RNA
<213>  Homo sapiens

<400>  58
ggauaucauc auauacugua agu                                               23

<210>  59
<211>  21
<212>  RNA
<213>  Homo sapiens

<400>  59
ugucaguuug ucaaauaccc c                                                 21

<210>  60
<211>  22
<212>  RNA
<213>  Homo sapiens

<400>  60
uucaccaccu ucuccaccca gc                                                22

<210>  61
<211>  22
<212>  RNA
<213>  Homo sapiens

<400>  61
ggauaucauc auauacugua ag                                                22

<210>  62
<211>  20
<212>  RNA
<213>  Homo sapiens

<400>  62

ugucaguuug ucaaauaccc                                                    20

<210>  63
<211>  19
<212>  RNA
<213>  Homo sapiens

<400>  63
aucacauugc cagggauuu                                                     19

<210>  64
<211>  19
<212>  RNA
<213>  Homo sapiens

<400>  64
ugucaguuug ucaaauacc                                                     19

<210>  65
<211>  18
<212>  RNA
<213>  Homo sapiens

<400>  65
accccacucc ugguacca                                                      18

<210>  66
<211>  20
<212>  RNA
<213>  Homo sapiens

<400>  66
ugggaucucc ggggucuugg                                                    20

<210>  67
<211>  31
<212>  RNA
<213>  Homo sapiens

<400>  67
ccauguuggu caggcugguc uugaacuccu g                                       31

<210>  68
<211>  15
<212>  RNA
<213>  Homo sapiens

<400>  68
gagggaacgu gagcu                                                         15

<210>  69
<211>  22
<212>  RNA
<213>  Homo sapiens

<400>  69

ugagguagua guuuguacag uu                                                22

<210>  70
<211>  23
<212>  RNA
<213>  Homo sapiens

<400>  70
uaaggugcau cuagugcaga uag                                               23

<210>  71
<211>  21
<212>  RNA
<213>  Homo sapiens

<400>  71
uaaagugcug acagugcaga u                                                 21

<210>  72
<211>  23
<212>  RNA
<213>  Homo sapiens

<400>  72
ugugcaaauc caugcaaaac uga                                               23

<210>  73
<211>  22
<212>  RNA
<213>  Homo sapiens

<400>  73
uggauuucuu ugugaaucac ca                                                22

<210>  74
<211>  17
<212>  RNA
<213>  Homo sapiens

<400>  74
guuugucaaa uacccca                                                      17

<210>  75
<211>  23
<212>  RNA
<213>  Homo sapiens

<400>  75
aaugacacga ucacucccgu uga                                               23

<210>  76
<211>  18
<212>  RNA
<213>  Homo sapiens

<400>  76

aggaggagga ggaggacg                                                    18

<210>  77
<211>  20
<212>  RNA
<213>  Homo sapiens

<400>  77
agaguggcgc agcggaagcg                                                  20

<210>  78
<211>  22
<212>  RNA
<213>  Homo sapiens

<400>  78
aagcugccag uugaagaacu gu                                               22

<210>  79
<211>  15
<212>  RNA
<213>  Homo sapiens

<400>  79
cacaaccagu uacca                                                       15

<210>  80
<211>  17
<212>  RNA
<213>  Homo sapiens

<400>  80
ggcuguagug cgcuaug                                                     17

<210>  81
<211>  35
<212>  RNA
<213>  Homo sapiens

<400>  81
gccugagguc uacugcugcc uuauccagag cugcc                                 35

<210>  82
<211>  20
<212>  RNA
<213>  Homo sapiens

<400>  82
ucccccaggu gugauucuga                                                  20

<210>  83
<211>  16
<212>  RNA
<213>  Homo sapiens

<400>  83

cuuaugcagg aggacc                                                    16

<210>  84
<211>  22
<212>  RNA
<213>  Homo sapiens

<400>  84
aaaagcuggg uugagagggc ga                                              22

<210>  85
<211>  15
<212>  RNA
<213>  Homo sapiens

<400>  85
agagcagaag ggaag                                                      15